(19) Europäisches Patentamt
European Patent Office
Office européen des brevets

(11) Publication number: **0 186 451 B1**

(12) # EUROPEAN PATENT SPECIFICATION

(45) Date of publication of patent specification: **01.02.89**

(51) Int. Cl.[4]: **C 07 C 43/225**, C 07 C 43/12, C 07 C 41/22

(21) Application number: **85309287.2**

(22) Date of filing: **19.12.85**

(54) **Preparation of alkoxy halides.**

(30) Priority: **20.12.84 GB 8432276**

(43) Date of publication of application: **02.07.86 Bulletin 86/27**

(45) Publication of the grant of the patent: **01.02.89 Bulletin 89/05**

(84) Designated contracting states: **BE CH DE FR GB IT LI NL**

(56) References cited:
CH--A-- 385 386
FR--A-- 1 381 786
**JOURNAL OF ORGANIC CHEMISTRY, vol. 40, no. 1, 10th January 1975, pages 134-136; T.G. SQUIRES et al.: "Zinc chloride catalysis in the reaction of thionyl halides with aliphatic alcohols"**

(73) Proprietor: **The British Petroleum Company p.l.c.
Britannic House Moor Lane
London EC2Y 9BU (GB)**

(72) Inventor: **Hodgson, Philip Kenneth Gordon
The British Petroleum Comp. p.l.c. Chertsey Road
Sunbury-on-Thames Middlesex, TW16 7LN (GB)**
Inventor: **Stewart, Nevin John
The British Petroleum Comp. p.l.c. Chertsey Road
Sunbury-on-Thames Middlesex, TW16 7LN (GB)**

(74) Representative: **MacLeod, Malcolm et al
BP INTERNATIONAL LIMITED Patents Division Chertsey Road
Sunbury-on-Thames Middlesex TW16 7LN (GB)**

**EP 0 186 451 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a process for the preparation of alkyl alkoxy, aryl alkoxy or alkylaryl alkoxy halides which are suitable for use as intermediates in the preparation of surfactants.

Numerous attempts have been made to develop surfactant compositions for use in enhanced oil recovery and the patent literature is replete with descriptions of formulations, see for example, USP's 4,424,135, 4,159,037, 4,110,228, 4,066,124 and 4,018,278.

A useful summary of the art is given in Kirk-Othmer's Encyclopedia of Chemical Technology, Third Edition, Volume 17, pages 168-182. This indicates that most compositions contain (a) a main surfactant which is either a petroleum sulphonate or a synthetic hydrocarbyl sulphonate and (b) co-surfactants which include simple alcohols, ethoxylated alcohols and sulphated ethoxylated alcohols.

It has also been disclosed that alkyl and alkylaryl polyalkoxy alkylene sulphonates may be used as co-surfactants. These compounds are generally prepared by a three-stage process. In the first stage of a typical process an alcohol or alkyl phenol is condensed with an alkylene oxide in the presence of sodium or potassium hydroxide to form an alkoxylate. This is then halogenated by treatment with thionyl or sulphuryl chloride, usually in the absence of a catalyst. Finally the halide is converted to a sulphonate by reaction with sodium sulphite, again, usually in the absence of a catalyst.

We have now discovered that the use of a soluble inorganic catalyst in the second stage, the halogenation stage, results in a shorter reaction period and an increased yield of desired product when compared with the uncatalysed reaction.

Thus according to the present invention there is provided a process for the production of an alkyl alkoxy, aryl alkoxy or an alkylaryl alkoxy halide which process comprises reacting an alkyl, aryl or alkylaryl alkoxy alcohol of formula :

$$R—(OR^1)_mOH,$$

with a halogenating agent in the presence of a soluble compound of a metal of Groups IA or IIA of the Periodic Table as a catalyst, wherein R is an alkyl group containing 1 to 24, preferably 8 to 20, carbon atoms, a phenyl group or an alkyl aryl group of formula :

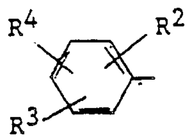

wherein $R^2$ is an alkyl group containing 1 to 24, preferably 8 to 20, carbon atoms and $R^3$ and $R^4$ are hydrogen atoms, or

$R^2$ and $R^3$ are both alkyl groups containing 1 to 12 carbon atoms and $R^4$ is a hydrogen atom, or

$R^2$, $R^3$ and $R^4$ are each alkyl groups wherein the sum of the number of carbon atoms in the groups is in the range 3 to 24,

$R^1$ is an alkylene group containing 2 or 3 carbon atoms, and

m is a number in the range 1 to 15, preferably 4 to 10.

Preferred halogenating agents are chlorinating agents such as thionyl chloride and sulphuryl chloride.

Suitable catalysts include the chlorides, hydroxides, alkoxides and alkanoates of lithium, magnesium, calcium, strontium and barium.

Other suitable catalysts include the above compounds of sodium, potassium, rubidium and caesium solubilised by a solubilising agent. Suitable agents include dipolar, aprotic solvents such as dimethyl sulphoxide, crown ethers, and polyalkoxy compounds containing three or more alkoxy groups per molecule such as dimethyl polyethylene glycol.

The reaction is suitably effected at a temperature in the range 30° to 120 °C, preferably in the range 75° to 85 °C.

Pressure is not a significant parameter and the reaction is therefore most conveniently carried out at atmospheric pressure.

The molar ratio of alkoxy alcohol to halogenating agent is suitably in the range 1 : 1 to 1 : 5, preferably 1 : 1 to 1 : 1.5.

The quantity of catalyst employed is preferably in the range 0.1 to 5 % by weight, expressed as a percentage by weight of the alkoxy alcohol.

The reaction may be effected in the presence or, preferably, the absence of a solvent. Suitable solvents include 1,2-dichloroethane, toluene and chloroform.

As a result of the reduced reaction time, the tendency of the polyoxyalkylene chain to cleave, liberating 1,4-dioxan in the case of a polyoxyethylene chain as noted by Robinson et al, J. Soc. Cosmet. Chem., 31, 329-337, is reduced. Thus both the yield and selectivity as indicated by the matching of the alkoxy distribution in the alcohol and the halide are improved and product contamination with unwanted by-product is reduced.

The invention is illustrated with reference to the following Examples. Examples 10 and 11 are not in accordance with the invention and are provided for comparison.

Example 1

Ethoxyalcohol $C_{18}H_{37}Ph(OCH_2CH_2)_5OH$ (41.80 g, 0.74M) was heated with stirring up to and at 80 °C with thionyl chloride (9.50 g, 0.080M) and anhydrous lithium chloride (0.41 g, 1 %).

$^{13}C$ NMR (Nuclear Magnetic Resonance) spectroscopy indicated quantitative chlorination after 0.25 hours at 80 °C and GLC (Gas Liquid Chromatography) indicated the production of 0.001 moles of 1,4-dioxan per mole ethoxyalcohol.

Example 2

Ethoxyalcohol $C_{18}H_{37}Ph(OCH_2CH_2)_5OH$ (78.20 g, 0.138M) was heated with stirring up to 80 °C with thionyl chloride (20.02 g, 0.168M) and lithium hydroxide monohydrate (0.78 g, 1 %).

$^{13}C$ NMR spectroscopy indicated quantitative chlorination when the reaction mixture had reached 80 °C and GLC indicated the production of 0.006 moles of 1,4-dioxan per mole ethoxyalcohol.

Example 3

Ethoxyalcohol $C_{18}H_{37}Ph(OCH_2CH_2)_5OH$ (58.65 g, 0.104M) was heated with stirring up to and at 80 °C with thionyl chloride (13.32 g, 0.112M) and calcium chloride (0.65 g, 1 %). $^{13}C$ NMR spectroscopy indicated quantitative chlorination after 1 hour at 80 °C and GLC indicated the production of 0.007 moles of 1,4-dioxan per mole ethoxyalcohol.

Example 4

Ethoxyalcohol $C_{18}H_{37}Ph(OCH_2CH_2)_5OH$ (75.34 g, 0.133M) was heated with stirring up to and at 80 °C with thionyl chloride (17.11 g, 0.144M) and anhydrous magnesium chloride (0.78 g, 1 %).

$^{13}C$ NMR spectroscopy indicated 98-99 % chlorination after 1 hour at 80 °C and GLC indicated the production of 0.017 moles of 1,4-dioxan per mole ethoxyalcohol.

Example 5

Ethoxyalcohol $C_{18}H_{37}Ph(OCH_2CH_2)_5OH$ (46.57 g, 0.082M) was heated with stirring up to and at 80 °C with thionyl chloride (10.57 g, 0.089M) and anhydrous strontium hydroxide (0.47 g, 1 %).

$^{13}C$ NMR spectroscopy indicated quantitative chlorination after 0.5 hours at 80 °C and GLC indicated the production of 0.007 moles of 1,4-dioxan per mole ethoxyalcohol.

Example 6

Ethoxyalcohol $C_{18}H_{37}Ph(OCH_2CH_2)_5OH$ (54.82 g, 0.097M) was heated with stirring up to and at 80 °C with thionyl chloride (12.45 g, 0.105M) and lithium hydroxide monohydrate (0.12 g, 0.2 %).

$^{13}C$ NMR spectroscopy indicated quantitative chlorination after 0.25 at 80 °C hours and GLC indicated the production of 0.006 moles of 1,4-dioxan per mole ethoxyalcohol.

Example 7

Ethoxyalcohol $C_{18}H_{37}Ph(OCH_2CH_2)_5OH$ (73.39 g, 0.130M) was heated with stirring up to and at 80 °C with thionyl chloride (16.66 g, 0.140M) and anhydrous lithium chloride (0.035 g, 0.05 %).

$^{13}C$ NMR spectroscopy indicated quantitative chlorination after 1 hour at 80 °C and GLC indicated the production of 0.017 moles of 1,4-dioxan per mole ethoxyalcohol.

Example 8

Ethoxyalcohol $C_{18}H_{37}Ph(OCH_2CH_2)_5OH$ (55.86 g, 0.099M) was heated with stirring up to and at 80 °C with thionyl chloride (12.69 g, 0.017M) and anhydrous lithium chloride (0.05 g, 0.1 %).

$^{13}C$ NMR spectroscopy indicated quantitative chlorination after 0.5 hours at 80 °C and GLC indicated the production of 0.016 moles of 1,4-dioxan per mole ethoxyalcohol.

Example 9

Ethoxyalcohol $C_{18}H_{37}Ph(OCH_2CH_2)_5OH$ (46.00 g, 0.081M) was heated with stirring up to and at 80 °C with thionyl chloride (10.45 g, 0.088M) and sodium chloride (0.46 g, 1 %) solubilised by 18-Crown-6 ether (2.09 g, 4.5 %).

$^{13}C$ NMR spectroscopy indicated quantitative chlorination after 1 hour at 80 °C and GLC indicated the production of 0.02 moles of 1,4-dioxan per mole ethoxyalcohol.

Exemple 10 (for comparison)

Ethoxyalcohol $C_{18}H_{37}Ph(OCH_2CH_2)_5OH$ (66.82 g, 0.118M) containing sodium hydroxide (0.75 g, 1 %) was heated with stirring up to and at 80 °C with thionyl chloride (17.41 g, 0.143M).

$^{13}C$ NMR spectroscopy indicated 90 % chlorination after 3 hours at 80 °C and GLC indicated the production of 0.07 moles of 1,4-dioxan per mole ethoxyalcohol.

Example 11 (for comparison)

Ethoxyalcohol $C_{18}H_{37}Ph(OCH_2CH_2)_5OH$ (61.11 g, 0.108M) was heated with stirring up to and at 80 °C with thionyl chloride (13.88 g, 0.117M).

$^{13}C$ NMR spectroscopy indicated quantitative conversion to ethoxychloride after 3 hours at 80 °C and GLC indicated the production of 0.07 moles of 1,4-dioxan per mole ethoxyalcohol.

It should be noted when comparing Examples 1 to 9 with 10 and 11 that addition of a soluble catalyst resulted in considerable increases to the chlorination rates in addition to reducing significantly the dioxan production.

**Claims**

1. A process for the production of an alkyl alkoxy, aryl alkoxy or an alkylaryl alkoxy halide which process comprises reacting an alkyl, aryl or alkylaryl alkoxy alcohol of formula :

$$R—(OR^1)_mOH,$$

with a halogenating agent wherein R is an alkyl group containing 1 to 24 carbon atoms, a phenyl group, or an alkyl aryl group of formula :

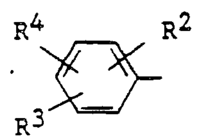

wherein $R^2$ is an alkyl group containing 1 to 24 carbon atoms and $R^3$ and $R^4$ are hydrogen atoms, or

$R^2$ and $R^3$ are both alkyl groups containing 1 to 12 carbon atoms and $R^4$ is a hydrogen atom, or

$R^2$, $R^3$ and $R^4$ are each alkyl groups wherein the sum of the number of carbon atoms in the groups is in the range 3 to 24 ;

$R^1$ is an alkylene group containing 2 or 3 carbon atoms, and

m is a number in the range 1 to 15,

characterised by the fact that the reaction is carried out in the presence of a soluble compound of a metal of Groups IA or IIA of the Periodic Table as a catalyst.

2. A process according to claim 1 wherein R is an alkyl group containing 8 to 20 carbon atoms, or $R^2$ is an alkyl group containing 8 to 20 carbon atoms and $R^3$ and $R^4$ are hydrogen atoms, and m is a number in the range 4 to 10.

3. A process according to either of the preceding claims wherein the halogenating agent is thionyl chloride or sulphuryl chloride.

4. A process according to any of the preceding claims wherein the catalyst is selected from the group consisting of the chlorides, hydroxides, alkoxides and alkanoates of lithium, magnesium, calcium, strontium and barium.

5. A process according to any of claims 1 to 3 wherein the catalyst is selected from the group consisting of the chlorides, hydroxides, alkoxides and alkanoates of sodium, potassium, rubidium and caesium solubilised by a solubilising agent.

6. A process according to claim 5 wherein the solubilising agent is selected from the group consisting of dipolar aprotic solvents, crown ethers and polyalkoxy compounds containing three or more alkoxy groups per molecule.

7. A process according to any of the preceding claims wherein the halogenation reaction is effected at a temperature in the range 30° to 120 °C.

8. A process according to any of the preceding claims wherein the molar ratio of alkoxy alcohol to halogenating agent is in the range 1 : 1 to 1 : 5.

9. A process according to any of the preceding claims wherein the quantity of catalyst employed is in the range 0.1 % to 5 % by weight, expressed as a percentage by weight of the alkoxy alcohol.

10. A process according to any of the preceding claims wherein the reaction is carried out in the absence of a solvent.

## Patentansprüche

1. Verfahren zur Herstellung eines Alkylalkoxy-, Arylalkoxy- oder Alkylarylalkoxyhalogenids, umfassend die Umsetzung eines Alkyl-, Aryl- oder Alkylarylalkoxyalkohols der Formel

$$R-(OR^1)_mOH$$

mit einem Halogenierungsmittel,
worin
R eine Alkyl-Gruppe mit 1 bis 24 Kohlenstoff-Atomen, eine Phenyl-Gruppe oder eine Alkylaryl-Gruppe der Formel

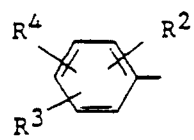

ist, in der
$R^2$ eine Alkyl-Gruppe mit 1 bis 24 Kohlenstoff-Atomen ist und
$R^3$ und $R^4$ Wasserstoff-Atome sind oder
$R^2$ und $R^3$ beide Alkyl-Gruppen mit 1 bis 12 Kohlenstoff-Atomen sind und
$R^4$ ein Wasserstoff-Atom ist oder
$R^2$, $R^3$ und $R^4$ jeweils Alkyl-Gruppen sind, wobei die Summe der Zahl der Kohlenstoff-Atome in den Gruppen im Bereich von 3 bis 24 liegt,
$R^1$ eine Alkylen-Gruppe mit 2 oder 3 Kohlenstoff-Atomen ist und
m eine Zahl im Bereich von 1 bis 15 ist, dadurch gekennzeichnet, daß die Reaktion in Gegenwart einer löslichen Verbindung eines Metalls der Gruppen IA oder IIA des Periodensystems als Katalysator durchgeführt wird.

2. Verfahren nach Anspruch 1, worin
R eine Alkyl-Gruppe mit 8 bis 20 Kohlenstoff-Atomen ist oder
$R^2$ eine Alkyl-Gruppe mit 8 bis 20 Kohlenstoff-Atomen ist und
$R^3$ und $R^4$ Wasserstoff-Atome sind und
m eine Zahl im Bereich von 4 bis 10 ist.

3. Verfahren nach irgendeinem der vorhergenden Ansprüche, worin das Halogenierungsmittel Thionylchlorid oder Sulfurylchlorid ist.

4. Verfahren nach einem der vorhergenden Ansprüche, worin der Katalysator aus der aus den Chloriden, Hydroxiden, Alkoxiden und Alkanoaten von Lithium, Magnesium, Calcium, Strontium und Barium bestehenden Gruppe ausgewählt ist.

5. Verfahren nach einem der Ansprüche 1 bis 3, worin der Katalysator aus der aus den Chloriden, Hydroxiden, Alkoxiden und Alkanoaten von Natrium, Kalium, Rubidium und Caesium, die mittels eines Lösungsvermittlers solubilisiert wurden, bestehenden Gruppe ausgewählt ist.

6. Verfahren nach Anspruch 5, worin der Lösungsvermittler aus der aus dipolaren aprotischen Lösungsmitteln, Kronenethern und Polyalkoxy-Verbindungen mit drei oder mehr Alkoxy-Gruppen im Molekül bestehenden Gruppe ausgewählt ist.

7. Verfahren nach irgendeinem der vorhergenden Ansprüche, worin die Halogenierungsreaktion bei einer Temperatur im Bereich von 30 °C bis 120 °C durchgeführt wird.

8. Verfahren nach irgendeinem der vorhergenden Ansprüche, worin das Stoffmengen-Verhältnis (« Mol-Verhältnis ») Alkoxyalkohol zu Halogenierungsmittel im Bereich von 1 : 1 bis 1 : 5 liegt.

9. Verfahren nach irgendeinem der vorhergenden Ansprüche, worin die Menge des eingesetzten Katalysators im Bereich von 0,1 bis 5 Gew.-% des Alkoxyalkohols liegt.

10. Verfahren nach irgendeinem der vorhergenden Ansprüche, worin die Reaktion in Abwesenheit eines Lösungsmittels durchgeführt wird.

## Revendications

1. Procédé pour la production d'un halogénure d'alcoxy alkyle, d'alcoxy aryle ou d'alcoxy alkylaryle, ce procédé comprenant la réaction d'un alcool alcoxy alkylique, alcoxy arylique ou alcoxy alkylarylique de formule :

$$R-(OR^1)_mOH$$

avec un agent d'halogénation, R représentant un groupe alkyle contenant 1 à 24 atomes de carbone, un groupe de phényl ou un groupe alkylaryle de formule

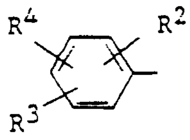

dans laquelle $R^2$ représente un groupe alkyle contenant 1 à 24 atomes de carbone et $R^3$ et $R^4$ représentent des atomes d'hydrogène, ou bien

$R^2$ ou $R^3$ représentent chacun des groupes alkyles contenant 1 à 12 atomes de carbone et $R^4$ représente un atome d'hydrogène, ou bien

$R^2$, $R^3$ et $R^4$ représentent chacun des groupes alkyles, la somme du nombre des atomes de carbone présents dans les groupes se situant entre 3 et 24 ;

$R^1$ représente un groupe alkylène contenant 2 ou 3 atomes de carbone, et

m est un nombre se situant entre 1 et 15, procédé caractérisé par le fait que l'on conduit la réaction en présence d'un composé soluble d'un métal des groupes IA ou IIA du Tableau Périodique des Eléments, à titre de catalyseur.

2. Procédé selon la revendication 1, dans lequel R représente un groupe alkyle contenant 8 à 20 atomes de carbone, ou bien $R^2$ représente un groupe alkyle contenant 8 à 20 atomes de carbone et $R^3$ et $R^4$ représentent des atomes d'hydrogène, et m est un nombre se situant entre 4 et 10.

3. Procédé selon l'une ou l'autre des revendications précédentes, dans lequel l'agent d'halogénation est le chlorure de thionyle ou le chlorure de sulfuryle.

4. Procédé selon l'une quelconque des revendications précédentes, dans lequel le catalyseur est choisi dans l'ensemble constitué par les chlorures, hydroxydes, alcoolates et alcanoates de lithium, de magnésium, de calcium, de strontium et de baryum.

5. Procédé selon l'une quelconque des revendications 1 à 3, dans lequel le catalyseur est choisi dans l'ensemble constitué par les chlorures, hydroxydes, alcoolates et alcanoates de sodium, de potassium, de rubidium et de césium, solubilisés par un agent de solubilisation.

6. Procédé selon la revendication 5, dans lequel l'agent de solubilisation est choisi dans l'ensemble constitué par les solvants aprotiques dipolaires, les éthers couronnes et les composés polyalcoxylés contenant trois ou plus de trois groupes alcoxy par molécule..

7. Procédé selon l'une quelconque des revendications précédentes, dans lequel on conduit la réaction d'halogénation en opérant à une température comprise entre 30° et 120 °C.

8. Procédé selon l'une quelconque des revendications précédentes, dans lequel le rapport molaire de l'alcoxy alcool à l'agent d'halogénation se situe entre 1 : 1 et 1 : 5.

9. Procédé selon l'une quelconque des revendications précédentes, dans lequel la quantité de catalyseur que l'on utilise se situe entre 0,1 % et 5 % en poids, cette quantité étant exprimée en pourcentage pondéral de l'alcoxy alcool.

10. Procédé selon l'une quelconque des revendications précédentes, dans lequel on conduit la réaction en l'absence d'un solvant.